# FASCICULE DE BREVET EUROPEEN

(11) **EP 0 946 486 B1**
(45) Date de publication et mention de la délivrance du brevet: **02.08.2006**
(21) Numéro de dépôt: 97947102.6
(22) Date de dépôt: 20.11.1997
(51) Int. Cl.: C07C 57/04, C07C 51/48

(54) **PURIFICATION DE L'ACIDE ACRYLIQUE OBTENU PAR OXYDATION CATALYTIQUE DU PROPYLENE**
REINIGUNG DER DURCH KATALYTISCHE PROPYLENOXIDATION ERHALTENE ACRYLSÄURE
PURIFICATION OF ACRYLIC ACID OBTAINED BY CATALYTIC OXIDATION OF PROPYLENE

(30) Priorité: 25.11.1996 FR 9614397
(43) Date de publication de la demande: 06.10.1999
(73) Titulaire: Arkema France, 92800 Puteaux (FR)
(72) Inventeur: FAUCONET, Michel, F-57730 Valmont (FR); ESCH, Marc, F-57800 Freyming Merlebach (FR); LAURENT, Denis, F-57500 Saint-Avold (FR)
(86) Numéro de dépôt international: PCT/FR1997/002092
(87) Numéro de publication internationale: WO 1998/023573

(56) Documents cités:
- DE-A- 2 449 780
- FR-A- 2 196 986

## Description

La présente invention concerne la purification de l'acide acrylique.

La principale voie de synthèse d'acide acrylique utilisée industriellement aujourd'hui est l'oxydation catalytique du propylène, qui génère intermédiairement l'acroléine. Cette réaction, qui a lieu en phase gazeuse, génère un flux gazeux contenant principalement, outre l'acide acrylique, des gaz incondensables : propylène non converti, azote, monoxyde et dioxyde de carbone, des composés organiques "légers", c'est-à-dire dont le point d'ébullition est inférieur à celui de l'acide acrylique : vapeur d'eau, acroléine non convertie, impuretés fabriquées par des réactions secondaires: formaldéhyde, acide acétique, et enfin, des composés lourds : anhydride maléique, furfuraldéhyde, benzaldéhyde, etc...

Les procédés de purification de ce gaz réactionnel, décrits dans la littérature, consistent à condenser ce mélange et à extraire les composés organiques par lavage à contre-courant à l'aide d'eau ou de solvants lourds.

Les procédés utilisant une absorption par l'eau présentent l'inconvénient d'extraire de manière peu sélective la quasi-totalité des produits organiques présents dans le mélange gazeux. La purification de la solution aqueuse ainsi constituée nécessite des séparations difficiles et coûteuses, par distillation et/ou extraction.

Dans le brevet français n° 1 558 432, est décrit un procédé qui consiste à absorber les composés organiques contenus dans le gaz de réaction à l'aide d'esters d'acides aliphatiques ou aromatiques à points d'ébullition élevés, ou de phosphate de tributyle ou de tricrésyle. A l'issue de cette étape d'absorption, les légers (acroléine, formaldéhyde) sont éliminés en tête d'une première colonne de distillation, et une deuxième colonne de distillation permet d'obtenir, en tête, une solution aqueuse d'acide acrylique plus concentrée que dans l'art antérieur. Toutefois, la purification ultérieure de la solution obtenue, qui contient encore de l'acide acétique et de l'eau, nécessite encore des séparations coûteuses.

Le procédé décrit dans le brevet français n° 2 002 126 apporte une amélioration, grâce à l'utilisation d'un mélange de fractions de points d'ébullition élevés, récupéré en pied des colonnes de purification des esters fabriqués à partir de l'acide acrylique, contenant principalement des maléates, acides polyacryliques, polyacrylates. Ce procédé permet de débarrasser en une seule étape, en tête d'une colonne de distillation, la plus grande part des composés de points d'ébullition faibles, tels que l'acroléine, le formaldéhyde, l'eau et l'acide acétique. Toutefois, ce procédé de fabrication d'esters acryliques est mal adapté à la production d'acide acrylique pur, notamment à cause de la présence, dans le mélange d'acide acrylique brut initial, des dérivés d'estérification recyclés à l'étape d'absorption.

Une amélioration est apportée dans le procédé utilisant une extraction à l'aide de solvants lourds hydrophobes, tel que décrit dans les brevets français n' 2 146 386, allemand n' 4 308 087 et européen n° 706 986, qui permet d'obtenir, à l'issue de l'étape d'extraction, une solution anhydre et débarrassée d'une partie substantielle des produits organiques légers qui constituaient le mélange gazeux initial (acroléine, formaldéhyde, acide acétique), facilitant ainsi sensiblement la purification ultérieure de l'acide acrylique.

Dans le brevet français n°2 146 386, est décrite l'utilisation d'un solvant hydrophobe ayant une température d'ébullition supérieure à 170°C sous pression atmosphérique et une viscosité inférieure à 10 est dans un intervalle de température de 30-80°C, et, en particulier, l'utilisation d'un mélange de diphényle (DP) et diphényléther (DPO) comme solvant d'absorption, avec notamment, environ 25% en poids de DP et 75% en poids de DPO. Ce mélange forme un eutectique (à une concentration massique de 26,5% de DP et 73,5% de DPO) qui présente l'avantage d'avoir un point de solidification (S = 12°C) plus bas que le DPO seul (F = 27°C).

Il est bien connu que la distillation de monomères acryliques, et en particulier de l'acide acrylique, qui polymérisent facilement sous l'action de radicaux formés par exemple par effet thermique, nécessite de mettre en oeuvre des inhibiteurs de polymérisation, particulièrement pendant les étapes de distillation. Les composés typiquement utilisés dans ce but sont, par exemple, les dérivés phénoliques comme l'hydroquinone ou l'éther méthylique de l'hydroquinone (p-méthoxyphénol), ou la phénothiazine et ses dérivés, ou les dérivés de la famille des thiocarbamates, ou les composés à groupements nitroso, ou les quinones, ou encore les amines aromatiques.

Dans les exemples du brevet français n° 2 146 386, l'inhibiteur mis en jeu est la phénothiazine. Malheureusement, l'application du procédé décrit selon ce brevet entraîne un encrassement des équipements de distillation par des impuretés polymériques, qui finissent par provoquer des bouchages de l'installation.

Pour réduire ce problème, le brevet allemand n° 4 308 087 décrit l'utilisation d'un solvant d'absorption constitué par le mélange eutectique de DP et DPO, et du diméthylphtalate (DMP), à une teneur de 0,1 à 25% en poids dans le mélange total.

Le brevet européen n' 706 986 décrit dans ses exemples l'utilisation du mélange eutectique DP + DPO et d'un mélange de 80% en poids de ce binaire + 20% en poids de DMP.

Les mélanges utilisant le DMP présentent deux inconvénients majeurs :
- le DMP est nettement moins hydrophobe que le mélange DP + DPO. En présence de 10% en poids d'acide acrylique dans le solvant (conditions approximatives du procédé), la solubilité de l'eau dans le mélange DP + DPO est d'environ 0,1% en poids contre environ 4% pour le DMP. Or, la présence d'eau dans le procédé est inévitable, puisque cette impureté est générée dans la réaction initiale d'oxydation du propylène conduisant à l'acide acrylique. Il s'ensuit que le flux récupéré en pied de colonne d'absorption est beaucoup plus riche en eau et en acide acétique puisque l'eau, par affinité, augmente l'absorption de l'acide acétique dans le flux de solvant, ce qui complique les purifications ultérieures. Ce désavantage se retrouve naturellement dans le procédé décrit dans le brevet français n° 2 196 986, qui décrit l'utilisation d'esters carboxyliques en tant que solvants d'absorption, puisque selon ce procédé, l'acide acétique et l'eau sont absorbés en même temps que l'acide acrylique ;
- en présence d'eau, le DMP subit une réaction secondaire d'hydrolyse, favorisée par le niveau thermique de l'étape d'absorption. Cette réaction parasite conduit à la formation d'impuretés nouvelles, comme l'anhydride phtalique et le méthanol, qui réagit avec l'acide acrylique pour former l'ester (acrylate de méthyle) correspondant. Outre les difficultés supplémentaires liées à la séparation de ces impuretés, cette réaction d'estérification génère une perte d'acide acrylique.

Le même désavantage, lié à la réaction parasite d'hydrolyse du solvant, existe pour tous les composés hydrolysables, en particulier ceux de la famille des esters. Le procédé décrit dans le brevet n° 2 196 986, qui revendique l'utilisation, comme solvants d'absorption, d'esters carboxyliques lourds ayant un point de fusion inférieur à 30°C et un point d'ébullition supérieur à 160°C, présente donc ce même inconvénient.

Un autre inconvénient du mélange DP + DPO est le fait qu'il soit constitué de deux composés différents, qui possèdent une température d'ébullition légèrement différente :
- DP : Eb = 255°C sous pression atmosphérique,
- DPO : Eb = 258°C sous pression atmosphérique.

Dans un procédé tel que décrit dans les brevets français n' 2 146 368 et allemand n' 4 308 087, le solvant lourd hydrophobe est recyclé à l'étape d'absorption, après élimination des impuretés légères absorbées, récupération de l'acide acrylique, purge des composés lourds intermédiaires (dont la température d'ébullition est comprise entre celle du solvant et celle de l'acide acrylique), et purge des composés lourds (impuretés et inhibiteurs de polymérisation qui ont un point d'ébullition supérieur à celui du solvant).

L'élimination des composés lourds intermédiaires et des composés plus lourds que le solvant peut être réalisée par lavage à l'eau ou par distillation.

Le premier procédé présente l'inconvénient de générer un effluent aqueux riche en pollution organique, dont le solvant présent à sa limite de solubilité dans l'eau. Par ailleurs, certains constituants peu solubles dans l'eau sont imparfaitement éliminés par cette méthode, et peuvent s'accumuler dans la boucle du solvant recyclé.

Pour éviter un phénomène d'accumulation de ces stabilisants, qui sont introduits de manière continue dans les colonnes de distillation, le recyclage du solvant à l'étape d'absorption nécessite la purge d'une quantité d'inhibiteurs au moins égale à celle qui est introduite dans les colonnes. La plupart des inhibiteurs efficaces pour la purification des monomères sensibles à la polymérisation possédant une structure aromatique qui les rend très peu solubles dans l'eau, ce procédé par lavage à l'eau ne convient pas à leur élimination.

Le second procédé d'élimination des impuretés lourdes par distillation, tel que décrit dans le brevet français n' 2 146 386, nécessite deux étapes distinctes, qui sont mises en oeuvre après celle de la séparation de l'acide acrylique :
· une étape de distillation des impuretés lourdes intermédiaires (à point d'ébullition compris entre celui de l'acide acrylique et celui du solvant), dans laquelle on sépare, en tête d'une colonne, un mélange contenant les impuretés intermédiaires, dont la principale est l'anhydride maléique. L'inconvénient principal de cette séparation est que, pour atteindre une pureté suffisante de l'acide acrylique distillé dans la colonne de purification précédente, c'est-à-dire débarrassée de ses impuretés lourdes, on doit accepter qu'une partie de ce monomère se retrouve en fond de ladite colonne de purification. Dans la colonne de séparation des impuretés lourdes intermédiaires, l'acide acrylique contenu dans ce flux est éliminé avec les impuretés en question, engendrant une perte coûteuse. Le procédé décrit dans la demande de brevet français n' 95-08672 déposée le 18 juillet 1995 au nom de la Société déposante apporte une amélioration à ce problème, grâce à l'élimination des impuretés lourdes intermédiaires dans un flux soutiré latéralement dans la colonne, et la récupération, dans le but d'être recyclé, de l'acide acrylique dans le flux de tête de cette même colonne. Toutefois, les procédés décrits dans ces deux brevets (FR 2 146 386 et demande française n° 95-08672) présentent l'inconvénient, lorsque le solvant utilisé est un mélange de DP et de DPO, de provoquer un enrichissement progressif en composé le plus lourd (DPO) du solvant circulant dans la boucle de purification, à cause de la différence des températures d'ébullition des deux constituants. Pour maintenir une composition constante du solvant circulant dans la boucle de purification, afin de maintenir une efficacité d'absorption régulière et une température constante dans les colonnes, on est donc contraint de faire un ajout de la quantité adéquate du composé le plus léger (DP), ce qui représente une opération compliquée et coûteuse.
   Ce type d'inconvénient est encore plus gênant dans le cas de l'utilisation d'un mélange de DP + DPO et de DMP, tel que décrit dans le brevet allemand n° 4 308 087, puisque ce procédé revient à ajouter une troisième composé qui possède une température d'ébullition différente (DMP : Eb = 284'C).
· une étape de séparation des impuretés lourdes, dans laquelle on élimine les composés à température d'ébullition supérieure à celle du solvant en pied d'une colonne de distillation ou d'un évaporateur. Cette étape est particulièrement coûteuse en énergie, puisqu'elle nécessite de distiller un solvant à point d'ébullition élevé. Le principal objectif de cette étape est d'éliminer les inhibiteurs de polymérisation, qui sont introduits de manière continue dans chacune des colonnes et doivent être purgés pour éviter leur accumulation dans la boucle de solvant à recycler. Les stabilisants efficaces pour la purification des monomères acryliques, comme l'acide acrylique, présentent des points d'ébullition supérieurs à celui des constituants du mélange DP + DPO, et ne peuvent par conséquent être éliminés qu'en pied de l'équipement de distillation.

Enfin, un dernier inconvénient du mélange DP + DPO est que ce mélange liquide se solidifie à la température de +12°C, ce qui nécessite, pour éviter tout figeage lorsque la température extérieure est plus basse, de réchauffer les conduites, appareillages et bacs de stockage véhiculant le solvant avant recyclage à l'étape d'absorption. Compte tenu des quantités importantes de solvant nécessaires pour absorber l'acide acrylique, cet inconvénient peut présenter des coûts d'investissement et d'énergie non négligeables.

Compte tenu de ces divers inconvénients, liés à l'utilisation des solvants revendiqués dans l'art antérieur, la Société déposante a recherché d'autres solvants hydrophobes, ne présentant pas ces difficultés, pour l'absorption de l'acide acrylique.

De manière surprenante, la Société déposante a découvert que le ditolyléther sous la forme d'un isomère seul ou d'un mélange d'isomères, utilisé comme solvant hydrophobe aromatique pour l'absorption de l'acide acrylique, améliore les séparations des impuretés contenues dans le mélange réactionnel. En outre, il est apparu lors des expérimentations que certains stabilisants phénoliques plus légers que ce solvant présentaient une activité inhibitrice de polymérisation meilleure que les inhibiteurs décrits dans l'art antérieur (comme la phénothiazine dans le brevet français n' 2 146 386). En outre, ces composés peuvent être éliminés efficacement, en une seule étape moins coûteuse en énergie au lieu de deux étapes, avec les composés lourds intermédiaires.

La présente invention a donc d'abord pour objet un procédé de purification de l'acide acrylique obtenu par oxydation catalytique du propylène, suivant lequel on extrait l'acide acrylique par lavage à contre-courant des gaz de réaction par au moins un solvant lourd d'absorption hydrophobe, puis on récupère l'acide acrylique purifié à partir de la solution obtenue à l'issue de cette étape d'extraction, caractérisé par le fait qu'on utilise, comme solvant lourd d'absorption hydrophobe, le ditolyléther sous la forme d'un isomère seul ou d'un mélange d'isomères.

L'utilisation du ditolyléther sous la forme d'un isomère seul ou d'un mélange d'isomères présente les avantages suivants :
- un seul constituant (pas de problème de séparation par distillation) ;
- séparations facilitées des légers (principalement acide acétique) dans l'étape d'absorption-stripping et des lourds dans les colonnes suivantes ;
- point de figeage très bas (-54°C), ce qui évite tout problème de cristallisation par temps froid.

Conformément à un mode de réalisation particulièrement intéressant de la présente invention, on conduit la purification de l'acide acrylique après l'étape d'extraction en présence, lorsque des distillations sont en jeu, d'un inhibiteur de polymérisation ou d'un mélange d'inhibiteurs de polymérisation, plus léger que le ditolyléther , en particulier présentant un écart de point d'ébullition supérieur à 15°c, de préférence supérieur à 20°C, avec le ditolyléther. Ces inhibiteurs sont également appelés ci-après "inhibiteurs de polymérisation légers". On peut citer, à titre d'exemples de ces inhibiteurs légers, le p-méthoxyphénol, le 2,6-ditertiobutyl p-crésol et le 2,4-diméthyl 6-tertiobutyl phénol.

Le procédé de purification de l'acide acrylique selon l'invention est par ailleurs avantageusement caractérisé par le fait :
- que l'on conduit, dans une colonne de distillation (C3), une distillation du flux obtenu en pied de la colonne d'extraction (C1) dans laquelle est effectuée l'extraction de l'acide acrylique par lavage à contre-courant des gaz de réaction par le ditolyléther , ledit flux contenant le ditolyléther , l'acide acrylique recherché et des impuretés, principalement des impuretés dont les températures d'ébullition sont supérieures à celle de l'acide acrylique, ladite distillation étant effectuée dans des conditions telles que l'on obtienne un flux très pur d'acide acrylique en tête de ladite colonne (C3), en laissant passer de l'acide acrylique en pied ;
- que l'on envoie le flux de pied de la colonne (C3) en alimentation dans la partie inférieure d'une colonne de distillation (C4) de laquelle on soutire latéralement, sur un plateau situé entre l'alimentation et la tête de colonne, un flux riche en anhydride maléique et impuretés à températures d'ébullition situées entre celle de l'acide acrylique et celle du ditolyléther ou du plus léger des isomères du ditolyléther utilisés en mélange ;
- que l'on distille, en tête de la colonne (C4), un flux riche en acide acrylique, lequel peut avantageusement être renvoyé sur la colonne (C3) ; et
- qu'on récupère, en pied de ladite colonne (C4), un flux contenant dudit (ou desdits) solvant(s) lourd(s) et des impuretés lourdes, dont les températures d'ébullition sont supérieures à celle du ditolyléther ou du plus léger des isomères du ditolyléther utilisés en mélange, flux que l'on recycle en tête de la colonne (C1) pour l'extraction de l'acide acrylique contenu dans les gaz de réaction.

Avant d'adresser à la colonne (C3) le flux obtenu en pied de la colonne (C1), on peut avantageusement débarrasser ledit flux d'une partie de ses impuretés résiduelles légères, telles que l'acide acétique et l'eau, en tête d'une colonne (S1), qui peut fonctionner comme une colonne de distillation classique équipée d'un rebouilleur en pied, ou comme une colonne de stripping alimentée en pied par un gaz (air, gaz inerte ou mélange gazeux incondensé récupéré en tête de colonne (C1), ou leur combinaison). De manière préférentielle, le flux gazeux obtenu en tête de colonne (S1), qui contient encore de l'acide acrylique, est renvoyé dans la colonne (C1).

Conformément à des modes de réalisation particuliers du procédé selon la présente invention :
- on envoie le flux obtenu en pied de la colonne (C1), le cas échéant en pied de la colonne (S1), sur un plateau situé dans la moitié inférieure de la colonne (C3), et on recherche le point de fonctionnement de ladite colonne (C3) de façon à obtenir :
   - en tête, un flux composé :
      - majoritairement, soit au moins 95% en poids, d'acide acrylique ;
      - le reste étant constitué par les composés lourds : anhydride maléique, furfuraldéhyde, benzaldéhyde et traces du ditolyléther ; et
   - en pied, un flux composé de :
      - majoritairement, soit au moins 95% en poids, du ditolyléther et des impuretés lourdes ;
      - le reste étant constitué par de l'acide acrylique ;
- on soutire latéralement de la colonne (C4) le flux riche en anhydride maléique, impuretés lourdes et éventuellement inhibiteurs de polymérisation légers, sur un plateau intermédiaire, situé au-dessus de l'alimentation entre le quart inférieur et le quart supérieur de cette colonne, à une température choisie de façon à obtenir un flux d'une concentration au moins égale à 20% en poids en impuretés à températures d'ébullition comprises entre celle de l'acide acrylique et celle du ditolyléther ou du plus léger des isomères du ditolyléther utilisés en mélange ;
- on envoie le flux distillé en tête de la colonne (C4), qui contient :
   - majoritairement, soit au moins 90% en poids, d'acide acrylique ;
   - le reste étant constitué par des impuretés à températures d'ébullition supérieures ;
   dans la colonne (C3), au niveau de l'alimentation principale de cette colonne ou, de manière avantageuse, à un niveau situé au-dessus de cette alimentation ; et
- avant de recycler en tête de la colonne (C1) le flux obtenu en pied de colonne (C4), on débarrasse ledit flux ou une partie dudit flux de ses impuretés lourdes. à températures d'ébullition supérieures à celle du ditolyléther , par exemple par des techniques de distillation ou extraction à l'aide d'un solvant, utilisées éventuellement en complément d'un traitement thermique de dissociation mettant en jeu ou non un catalyseur.

Conformément à des modes de réalisation particuliers de la présente invention, on conduit la distillation :
- dans la colonne (C3), sous une pression de 2,66x10³⁻3,33x10⁴ Pa (20 - 250 mmHg), à une température de tête de 40 - 120°C et à une température de pied de 120 - 230°C ; et
- dans la colonne (C4), sous une pression de 2,66x10³⁻3,33x10⁴ Pa (20 - 250 mmHg), à une température de tête de 40 - 120°C, à une température de pied de 120 - 230°C et à une température de soutirage latéral de 40 - 180°C.

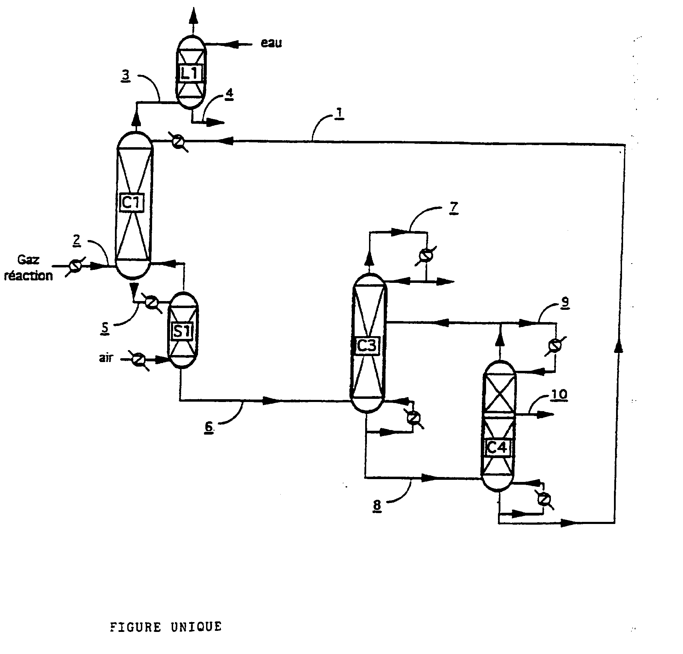

Les Exemples suivants illustrent la présente invention sans toutefois en limiter la portée. Dans ces exemples, les pourcentages sont donnés en poids. On a utilisé des colonnes de distillation qui sont installées selon la schéma de la Figure unique du dessin annexé et dont les caractéristiques sont indiquées ci-après. Dans le texte relatif à la description de ces exemples, on numérote les plateaux des colonnes à partir de la tête de colonne (plateau 0) jusqu'en pied de colonne (plateau n).

### . Colonne C1 :

Cette colonne, de 3 m de hauteur et de 38 mm de diamètre, garnie d'éléments de remplissage de type "multiknit", est alimentée :
- en tête, par un courant (1) de solvant d'absorption, pur ou récupéré en pied de colonne (C4) ; et
- en pied, par le courant (2) de gaz réactionnel d'oxydation catalytique du propylène, préalablement refroidi à la température désirée.

En tête de colonne (C1), on obtient un flux (3) riche en composés légers (gaz incondensables, acroléine, formaldéhyde, eau, et acide acétique).

Le flux gazeux (3) est lavé par de l'eau introduite à contre-courant dans une colonne de lavage (L1). Un bilan analytique des composés organiques présents dans le flux (3) est réalisé sur le flux (4) obtenu en pied de colonne (L1).

### . Colonne S1

Le but de la colonne (S1) est d'éliminer les composés légers présents dans le pied de la colonne (C1) (eau, acide acétique, acroléine,...). Cette colonne, de hauteur 1 m et de diamètre 38 mm, est garnie d'éléments de remplissage de type multiknit. Elle est alimentée en tête par le flux de pied de colonne C1, qui peut être préalablement réchauffé à travers un échangeur, et en pied par un débit contrôlé d'air, dont la température est régulée en passant à travers un échangeur. Le flux gazeux obtenu en tête de colonne (S1) est envoyé dans la colonne (C1), au niveau de l'alimentation des gaz réactionnels dans cette dernière.

En pied de colonne (S1), le flux (6) récupéré constitue une solution dans le solvant d'absorption de l'acide acrylique avec une faible partie des impuretés légères (acide acétique) et des impuretés lourdes présentes initialement dans le gaz réactionnel issus du réacteur d'oxydation : anhydride maléique, furfuraldéhyde, benzaldéhyde, composés d'addition sur l'acide acrylique, inhibiteurs.

Les colonnes (C1), (S1) et (L1) fonctionnent sous pression atmosphérique.

### · Colonne C3 :

Cette colonne adiabatique, garnie de 12 plateaux perforés, d'efficacité 9 plateaux théoriques, est alimentée en fond de colonne par le flux (6) obtenu en pied de colonne (S1). La colonne est équipée en partie inférieure d'un bouilleur à thermosiphon chauffé électriquement. Une partie du distillat (7) récupéré, après condensation dans un réfrigérant refroidi à 18°C, est recyclée en tête de colonne pour assurer le reflux. Une injection d'inhibiteur de polymérisation, en solution dans l'acide acrylique, est assurée en tête de condenseur. L'ensemble fonctionne sous un vide régulé à 1,33 x 10⁴ Pa(100 mm Hg).

### · Colonne C4 :

Cette colonne est constituée de 9 plateaux perforés avec surverse. L'alimentation, par un flux (8) obtenu en pied de colonne (C3), est envoyée en pied de cette colonne, au niveau du bouilleur à thermosiphon chauffé par résistances électriques. Une partie du distillat (9) condensé est renvoyée en tête de colonne pour assurer le reflux. Au niveau du 5ème plateau, un système permet de soutirer en (10) une partie de la phase liquide condensée. Ce système est muni d'une vanne dont l'ouverture est commandée automatiquement lorsque la température mesurée sur ce plateau atteint la consigne de température fixée.

Les Exemples suivants illustrent la présente invention sans toutefois en limiter la portée. Dans les exemples, les pourcentages des proportions sont en poids.

### EXEMPLES

### EXEMPLE 1 :

Le mélange gazeux réactionnel (2) obtenu en sortie de réacteurs d'oxydation catalytique du propylène est refroidi à travers un échangeur à une température de 160°C, puis envoyé, à un débit de 2160 g/h, en pied de la colonne (C1). Au même niveau, la colonne C1 reçoit le flux gazeux provenant de la tête de colonne S1.

A contre-courant, on alimente en tête de colonne (C1) (flux 1) 3680 g/h de ditolyléther à une température de 50°C. Le ditolyléther mis en oeuvre se présente sous la forme du mélange d'isomères ayant la composition suivante :

| | |
|---|---|
| . 2,2'-diméthyl-diphényl éther (ortho-ortho) | 1,9% |
| . 2,3'-diméthyl-diphényl éther (ortho-méta) | 24,8% |
| . 2,4'-diméthyl-diphényl éther (ortho-para) | 14,0% |
| . 3,3'-diméthyl-diphényl éther (méta-méta) | 30,0% |
| . 3,4'-diméthyl-diphényl éther (méta-para) | 24,9% |
| . 4,4'-diméthyl-diphényl éther (para-para) | 4,5% |

Le mélange liquide recueilli en pied de colonne (C1) (5) est ensuite envoyé en tête de la colonne (S1), à une température régulée à 120°C.

En pied de colonne (S1), on introduit un débit d'air de 580 1/h à une température de 50°C. Le flux gazeux sortant en tête de colonne (S1) est envoyé en pied de colonne (C1), au niveau d'alimentation des gaz réactionnels dans cette colonne. Le mélange brut obtenu en pied de colonne (6) est constitué principalement d'acide acrylique (7,73%), du solvant d'absorption et de faibles quantités de composés plus lourds que l'acide acrylique (anhydride maléique, furfuraldéhyde, benzaldéhyde, ...). Dans ce milieu, la teneur en composés légers est particulièrement faible (acide acétique : 0,011% ; eau : 0,007%).

Après une première condensation du flux gazeux (3) sortant en tête de colonne (C1), les vapeurs incondensées sont envoyées en pied de la colonne de lavage à l'eau, à contre-courant du flux aqueux (2750 g/h). La solution aqueuse obtenue en pied de (L1) (4) titre 0,026% d'acide acrylique et 0,345% d'acide acétique.

Dans ces conditions, les performances du système (C1)-(S1) sont telles que le taux d'absorption de l'acide acrylique atteint 99,8% et le taux d'élimination d'acide acétique 96,4%.

### EXEMPLE 2 :

Un mélange brut (6) obtenu en pied de colonne (S1), contenant principalement, outre le solvant ditolyléther, 10,12% d'acide acrylique et 0,06% d'anhydride maléique est envoyé avec un débit de 1166 g/h en pied de la colonne (C3), à une température de 90°C. La pression en tête de colonne (C3) est régulée à 1,33 x 10⁴ (100 mmHg). Le chauffage du bouilleur est réglé de façon à maintenir une température de 170°C en pied de colonne (C3). La température mesurée en tête de colonne, au niveau du plateau supérieur, est de 80,5°C. Une solution à 1,5% de p-méthoxyphénol dans l'acide acrylique est injectée à un débit de 20,2 g/h en tête du condenseur. Une partie du distillat (7), après condensation (115 g/h), est renvoyé en tête de la colonne. Le distillat recueilli (127 g/h) présente une excellente pureté en composés plus lourds que l'acide acrylique (0,03% d'anhydride maléique, 0,017% de furfuraldéhyde, et moins de 0,001% de benzaldéhyde et de ditolyléther). Le flux (8) obtenu en pied de colonne (1058 g/h) est essentiellement constitué de ditolyléther, et titre également 0,55% d'acide acrylique, 0,06% d'anhydride maléique.

### EXEMPLE 3 :

Un flux (8) récupéré en pied de colonne (C3), qui contient, outre le ditolyléther et les lourds, 0,145% d'acide acrylique et 0,07% de p-méthoxyphénol, est envoyé (1000 g/h) à une température de 160°C en pied de colonne (C4), qui fonctionne sous une pression de 6,66 x 10³ Pa (50 mmHg). La température au niveau du bouilleur est régulée à 191°C, et la température de consigne de soutirage au niveau du 5^{ème} plateau est fixée à 140°C. Le produit distillé en tête de colonne (1,45 g/h), destiné à être renvoyé en alimentation de colonne (C3), est composé de 96,8% d'acide acrylique, 3,2% d'anhydride maléique et ne contient aucune trace de solvant d'absorption. Le flux (10) soutiré en soutirage latéral (0,6 g/h) contient 1,47% d'acide acrylique, 54% d'anhydride maléique, 30% de solvant d'absorption et 10,8% de p-méthoxyphénol. Enfin, le mélange (1) obtenu en pied de colonne, destiné à être recyclé en tête de colonne (C1), est principalement constitué du solvant d'absorption et des produits lourds d'addition sur l'acide acrylique, sans aucune trace d'acide acrylique (inférieur à 0,005%) avec de faibles teneurs d'anhydride maléique (0,019%).

On remarquera, dans cet exemple, que le taux d'élimination de l'anhydride maléique dans le flux soutiré latéralement dans la colonne atteint 57%, et que la perte d'acide acrylique ne dépasse pas 0,6% de la teneur de ce monomère dans le flux d'alimentation. En outre, le soutirage latéral permet d'éliminer 10% du p-méthoxyphénol présent dans l'alimentation de la colonne, et ainsi de déconcentrer le solvant récupéré en pied de (C4), qui est destiné à être recyclé en tête de (C1).

### EXEMPLE 1-bis (comparatif) :

Dans les mêmes conditions que l'Exemple 1, on envoie, à une température de 50°C, 3930 g/h d'un solvant (1) constitué d'un mélange eutectique de diphényle (23,5%) et de diphényléther (73,5%) en tête de colonne C1, et en pied de cette même colonne, un gaz réactionnel issu d'une réaction catalytique d'oxydation du propylène. Ce mélange gazeux (2) est de composition identique à celle de l'Exemple 1, et alimente la colonne (C1) à la même température de 160°C. Cette colonne reçoit en outre, en pied, le flux gazeux issu de la tête de colonne (S1).

Le liquide obtenu en pied de colonne (C1) (5) alimente la colonne (S1) en tête de celle-ci, à une température de 120°C. En pied de colonne (S1), on envoie 600 1/h d'air préchauffé à 50°C.

Dans ces conditions, le mélange brut (6) obtenu en pied de colonne (S1) titre 5,76% d'acide acrylique, 0,01% d'acide acétique et 0,022% d'eau.

En tête de colonne (C1), le flux gazeux (3) est refroidi dans un réfrigérant, puis envoyé dans la colonne de lavage, qui reçoit en tête un débit de 2300 g/h d'eau. Le mélange (4) du condensat et du flux aqueux obtenu en pied de colonne de lavage titre 2,32% d'acide acrylique et 0,55% d'acide acétique.

Le taux d'élimination d'acide acétique dans l'ensemble (C1)-(S1) est de 95%, mais le taux d'absorption d'acide acrylique reste limité à 81,8% (soit une perte de 19,2%).

### EXEMPLE 2-bis (comparatif) :

En pied de la colonne (C3), qui travaille sous une pression de 1,33 x 10⁴ Pa (100 mmHg), on envoie, à une température de 90°C et avec un débit de 1164 g/h, un mélange de pied de colonne (S1) (6) contenant principalement le solvant, qui est un mélange eutectique composé de 73,5% de diphényloxyde et 26,5% de diphényle, avec en outre 9,5% d'acide acrylique et 0,06% d'anhydride maléique. La température de pied de colonne est régulée à 160°C. Une solution de p-méthoxyphénol (1,5%) dans l'acide acrylique est injectée à un débit de 23,2 g/h en tête du condenseur. Une partie du distillat (7), après condensation (115 g/h), est renvoyée en tête de la colonne. Les teneurs principales en composés lourds dans le distillat recueilli (126 g/h) sont de 0,05% d'anhydride maléique, 0,03% de furfuraldéhyde et 0,02% de benzaldéhyde. Le flux (8) obtenu en pied de colonne (1064 g/h) est essentiellement constitué du solvant, et titre également 0,50% d'acide acrylique, 0,06% d'anhydride maléique.

Cet exemple montre que, pour une même concentration d'acide acrylique en pied de colonne, la composition en lourds dans le flux d'acide acrylique distillé est plus élevée que dans l'Exemple 2.

### EXEMPLE 3-bis (comparatif)

En fond de colonne (C4), qui fonctionne sous une pression de 1,33 x 10⁴ Pa (100 mmHg), on envoie 1000 g/h d'un mélange récupéré en pied de colonne (C3), constitué principalement du solvant lourd d'absorption (mélange eutectique de diphényle et diphényléther), d'acide acrylique (0,69%), d'anhydride maléique (0,06%), d'hydroquinone (0,04%), ainsi que de faibles teneurs de composés lourds d'addition sur la double-liaison de l'acide acrylique. Ce mélange est préchauffé à une température de 180°C avant d'être envoyé dans le bouilleur de la colonne. La température dans le bouilleur est régulée à 182°C. La température de consigne de soutirage du flux riche en composés lourds intermédiaires, au niveau du 5^{ème} plateau, est fixée à 135°C. On distille en tête de colonne, à une température de 84°C, un flux (6,4 g/h) constitué de 97,1% d'acide acrylique, 2,49% d'anhydride maléique et 0,07% de solvant d'absorption. Le flux soutiré latéralement au niveau du 5^{ème} plateau (0,5 g/h) est composé de 62% d'anhydride maléique, 29% de solvant lourd d'absorption et 5,3% d'acide acrylique. Enfin, en pied de colonne, on soutire un mélange constitué essentiellement du solvant lourd d'absorption et des composés lourds d'addition sur l'acide acrylique, ainsi que 0,02% d'acide acrylique, 0,016% d'anhydride maléique et 0,038% d'hydroquinone.

Dans ces conditions, la perte d'acide acrylique dans les flux (10) et (1) atteint 3,3% de la quantité de monomère présente dans le flux (8) alimentant la colonne (C4). Le taux de réduction de l'anhydride maléique, soutiré latéralement, est de 48%. La totalité de l'inhibiteur hydroquinone se retrouve dans le flux de pied de (C4).

### EXEMPLE 4 (comparatif) :

En fond d'une colonne (C4) fonctionnant dans des conditions identiques à celles de l'Exemple Comparatif 3-bis, on envoie 1000 g/h d'un flux constitué d'un mélange eutectique de diphényle et de diphényléther contenant 0,31% d'acide acrylique, 0,29% d'anhydride maléique et 0,073% de p-méthoxyphénol. Le flux soutiré latéralement sur le plateau 5 (0,7 g/h), qui titre 55,5% d'anhydride maléique, ne contient que 0,28% de p-méthoxyphénol, et cet inhibiteur n'est présent qu'à l'état de traces (0,004%) dans le flux de tête (3,2 g/h). La quasi-totalité du p-méthoxyphénol est dans le flux obtenu en pied de colonne (976,6 g/h), qui titre 0,074% de ce stabilisant.

On remarque dans cet exemple que, contrairement à l'Exemple Comparatif 3-bis réalisé en milieu ditolyléther, le taux d'élimination de l'inhibiteur p-méthoxyphénol dans le flux de soutirage latéral est négligeable (0,3%), et ne suffit pas pour déconcentrer significativement en cet additif le solvant destiné à être recyclé en tête de (C1).

### EXEMPLE 5 :

Dans cet exemple, on évalue comparativement l'efficacité de différents inhibiteurs de polymérisation dans les conditions de distillation de la colonne (C3). Pour simuler la boucle de solvant qui est recyclée de nombreuses fois dans le procédé avec l'inhibiteur, les expériences ont été conduites avec recyclage total, dans le bac d'alimentation, des flux de tête et de pied de colonne préalablement rassemblés.

La colonne de distillation de hauteur 60 cm et de diamètre 38 mm, est équipée d'un garnissage de type multiknit et d'un bouilleur à thermosiphon chauffé par résistance électrique. Les essais sont réalisés sous une pression de 1,33 x 10⁴ Pa (100 mmHg). La recette d'alimentation (R1) de la colonne contient 4800 g d'un mélange composé de 10% d'acide acrylique et 0,05% de l'inhibiteur à tester, le complément étant le solvant, qui est le ditolyléther. On envoie ce mélange, préchauffé à travers un échangeur à une température de 100°C, à un débit de 1200 g/h, en pied de la colonne. La température dans le bouilleur est régulée à 170°C. La température de tête de colonne est de 83°C. Les vapeurs obtenues en tête de colonne sont condensées dans un réfrigérant vertical et le liquide condensé est recueilli dans une recette (R2). A partir de cette recette (R2), une pompe renvoie une partie du liquide condensé en tête de la colonne, avec un taux de reflux/coulage de 2/1. Un ajout de 24 g/h de l'inhibiteur à tester, en solution à 1,5% dans l'acide acrylique, est réalisé en tête de réfrigérant, pour stabiliser l'acide acrylique distillé. Le même débit de liquide est prélevé dans le flux sortant de la recette (R2) et éliminé de la boucle, pour maintenir un volume constant dans la boucle. Le flux de distillat restant est mélangé avec le flux obtenu en pied de colonne et renvoyé dans la recette d'alimentation (R1). On calcule que, du fait de l'ajout de stabilisant frais dans le distillat, la teneur initiale d'inhibiteur dans le flux d'alimentation (0,05%) augmente de 0,007% par heure.

Lorsque cette expérience est réalisée avec la phénothiazine comme inhibiteur, on observe, après 19 heures de fonctionnement, des dépôts polymériques bruns le long de la colonne et en tête de celle-ci, ainsi que dans la conduite renvoyant le reflux en tête de colonne. Ces dépôts ne cessent de s'accroître ensuite, jusqu'à un bouchage complet du garnissage après 65 heures de marche.

La même expérience, dans des conditions strictement identiques, a été réalisée avec des composés phénoliques relativement légers (dont le point d'ébullition est inférieur à 270°C). Les inhibiteurs suivants ont été testés indépendamment :
· le p-méthoxyphénol
· le 2,6-ditertiobutyl p-crésol
· le 2,4-diméthyl-6-tertiobutyl phénol

Dans les trois cas, aucune trace visible de dépôt n'a été observée à l'issue d'un temps de fonctionnement de 70 heures.

## Revendications

1. Procédé de purification de l'acide acrylique obtenu par oxydation catalytique du propylène, suivant lequel on extrait l'acide acrylique par lavage à contre-courant des gaz de réaction par au moins un solvant lourd d'absorption hydrophobe, puis on récupère l'acide acrylique purifié à partir de la solution obtenue à l'issue de cette étape d'extraction, **caractérisé par le fait qu'**on utilise, comme solvant lourd d'absorption hydrophobe, le ditolyléther sous la forme d'un isomère seul ou d'un mélange d'isomères.

2. Procédé selon la revendication 1, **caractérisé par le fait qu'**on conduit la purification de l'acide acrylique après l'étape d'extraction en présence, lorsque des distillations sont en jeu, d'un inhibiteur de polymérisation ou d'un mélange d'inhibiteurs de polymérisation, plus léger que le ditolyléther, en particulier présentant un écart de point d'ébullition supérieur à 15°C, de préférence supérieur à 20°C, avec le ditolyléther.

3. Procédé selon la revendication 2, **caractérisé par le fait que** le ou les inhibiteurs de polymérisation sont choisis parmi le p-méthoxyphénol, le 2,6-ditertiobutyl p-crésol et le 2,4-diméthyl 6-tertiobutyl phénol.

4. Procédé selon l'une des revendications 1 à 3, **caractérisé par le fait :**
- **que** l'on conduit, dans une colonne de distillation (C3), une distillation du flux (6) obtenu en pied de la colonne d'extraction (C1) dans laquelle est effectuée l'extraction de l'acide acrylique par lavage à contre-courant des gaz de réaction par le ditolyléther, ledit flux (6) contenant le ditolyléther, l'acide acrylique recherché et des impuretés, principalement des impuretés dont les températures d'ébullition sont supérieures à celle de l'acide acrylique, ladite distillation étant effectuée dans des conditions telles que l'on obtienne un flux très pur d'acide acrylique (7) en tête de ladite colonne (C3), en laissant passer de l'acide acrylique en pied (8) ;
- **que** l'on envoie le flux de pied (8) de la colonne (C3) en alimentation dans la partie inférieure d'une colonne de distillation (C4) de laquelle on soutire latéralement, sur un plateau situé entre l'alimentation et la tête de colonne, un flux (10) riche en anhydride maléique et impuretés à températures d'ébullition situées entre celle de l'acide acrylique et celle du ditolyléther ou du plus léger des isomères du ditolyléther utilisés en mélange ;
- **que** l'on distille, en tête de la colonne (C4), un flux (9) riche en acide acrylique, lequel peut avantageusement être renvoyé sur la colonne (C3) ; et
- **qu'**on récupère, en pied de ladite colonne (C4), un flux (1) contenant dudit (ou desdits) solvant(s) lourd(s) d'extraction et des impuretés lourdes, dont les températures d'ébullition sont supérieures à celle du ditolyléther pu du plus léger des isomères du ditolyléther, utilisés en mélange flux que l'on recycle en tête de la colonne (C1) pour l'extraction de l'acide acrylique contenu dans les gaz de réaction.

5. Procédé selon la revendication 4, **caractérisé par le fait qu'**on débarrasse le flux obtenu en pied de la colonne (C1) d'une partie de ses impuretés résiduelles légères, telles que l'acide acétique et l'eau, en tête d'une colonne (S1), qui peut fonctionner comme une colonne de distillation classique équipée d'un rebouilleur en pied, ou comme une colonne de stripping alimentée en pied par un gaz, tel que l'air, un gaz inerte ou un mélange gazeux incondensé récupéré en tête de colonne (C1), ou leur combinaison, le flux gazeux obtenu en tête de colonne (S1) qui contient encore de l'acide acrylique étant avantageusement renvoyé dans la colonne.

6. Procédé selon l'une des revendications 4 et 5, **caractérisé par le fait que** l'on envoie le flux (6) obtenu en pied de la colonne (C1), le cas échéant en pied de la colonne (S1), sur un plateau situé dans la moitié inférieure de la colonne (C3), et que l'on recherche le point de fonctionnement de ladite colonne (C3) de façon à obtenir :
- en tête, un flux (7) composé
- majoritairement, soit au moins 95% en poids, d'acide acrylique ;
- le reste étant constitué par les composés lourds : anhydride maléique, furfuraldéhyde, benzaldéhyde et traces du ditolyléther ; et
- en pied, un flux (8) composé de :
- majoritairement, soit au moins 95% en poids, du ditolyléther et des impuretés lourdes ;
- le reste étant constitué par de l'acide acrylique.

7. Procédé selon l'une des revendications 4 à 6, **caractérisé par le fait que** l'on soutire latéralement de la colonne (C4) le flux (10) riche en anhydride maléique et impuretés lourdes sur un plateau intermédiaire, situé au-dessus de l'alimentation entre le quart inférieur et le quart supérieur de cette colonne, à une température choisie de façon à obtenir un flux (10) d'une concentration au moins égale à 20% en poids en impuretés à températures d'ébullition comprises entre celle de l'acide acrylique et celle du ditolyléther ou du plus léger des isomères du ditolyléther utilisés en mélange.

8. Procédé selon l'une des revendications 4 à 7, **caractérisé par le fait que** l'on envoie le flux (9) distillé en tête de la colonne (C4), qui contient :
- majoritairement, soit au moins 90% en poids, d'acide acrylique ;
- le reste étant constitué par des impuretés à températures d'ébullition supérieures,
à la colonne (C3), au niveau de l'alimentation principale de cette colonne ou à un niveau situé au-dessus de cette alimentation.

9. Procédé selon l'une des revendications 4 à 8, **caractérisé par le fait qu'**avant de recycler en tête de la colonne (C1) le flux (1) obtenu en pied de colonne (C4), on débarrasse ledit flux ou une partie dudit flux (en 11) de ses impuretés lourdes à températures d'ébullition supérieures à celle du ditolyléther, par exemple par des techniques de distillation ou extraction à l'aide d'un solvant, utilisées éventuellement en complément d'un traitement thermique de dissociation mettant en jeu ou non un catalyseur.

10. Procédé selon l'une des revendications 4 à 9, **caractérisé par le fait qu'**on conduit la distillation :
- dans la colonne (C3) sous une pression de 2,66 x 10³ - 3,33 x 10⁴ Pa, à une température de tête de 40 - 120°C et à une température de pied de 120 - 230°C ; et
- dans la colonne (C4) sous une pression de 2,66 x 10³ - 3,33 x 10⁴ Pa, à une température de tête de 40 - 120°C, à une température de pied de 120 - 230°C, et à une température de soutirage latéral de 40 - 180°C.

## Claims

1. Process for the purification of acrylic acid obtained by catalytic oxidation of propylene, according to which the acrylic acid is extracted by countercurrentwise washing of the reaction gases with at least one heavy hydrophobic absorption solvent and then the purified acrylic acid is recovered from the solution obtained on conclusion of this extraction stage, **characterized in that** use is made, as heavy hydrophobic absorption solvent, of ditolyl ether in the form of a single isomer or of a mixture of isomers.

2. Process according to Claim 1, **characterized in that** the purification of the acrylic acid is carried out after the extraction stage in the presence, when distillations are involved, of a polymerization inhibitor or of a mixture of polymerization inhibitors which is lighter than ditolyl ether, in particular exhibiting a difference in boiling point of greater than 15°C, preferably of greater than 20°C, with ditolyl ether.

3. Process according to Claim 2, **characterized in that** the polymerization inhibitor or inhibitors are chosen from p-methoxyphenol, 2,6-di-tert-butyl-p-cresol and 2,4-dimethyl-6-tert-butylphenol.

4. Process according to one of Claims 1 to 3, **characterized in that**:
- a distillation is carried out, in a distillation column (C3), of the flow (6) obtained at the bottom of the extraction column (C1), in which column acrylic acid is extracted by countercurrentwise washing of the reaction gases with ditolyl ether, the said flow (6) comprising ditolyl ether, the desired acrylic acid and impurities, mainly impurities with boiling temperatures greater than that of acrylic acid, the said distillation being carried out under conditions such that a very pure acrylic acid flow (7) is obtained at the top of the said column (C3), acrylic acid being allowed to pass into the bottom (8);
- the bottom flow (8) from the column (C3) is conveyed as feed into the lower part of a distillation column (C4), from the side of which column is drawn off, on a plate situated between the feed and the column top, a flow (10) rich in maleic anhydride and impurities with boiling temperatures situated between that of acrylic acid and that of ditolyl ether or of the lighter of the ditolyl ether isomers used as a mixture;
- a flow (9) rich in acrylic acid is distilled at the top of the column (C4), which flow can advantageously be conveyed to the column (C3); and
- a flow (1) comprising the said heavy extraction solvent(s) and heavy impurities with boiling temperatures greater than that of ditolyl ether or of the lighter of the ditolyl ether isomers used as a mixture is recovered at the bottom of the said column (C4), which flow is recycled at the top of the column (C1) for the extraction of acrylic acid present in the reaction gases.

5. Process according to Claim 4, **characterized in that** the flow obtained at the bottom of the column (C1) is freed from a portion of its residual light impurities, such as acetic acid and water, at the top of a column (S1) which can operate as a conventional distillation column equipped with a bottom reboiler or as a stripping column fed at the bottom with a gas, such as air, an inert gas or a non-condensed gas mixture recovered at the top of column (C1), or their combination, the gas flow obtained at the top of column (S1), which still comprises acrylic acid, advantageously being conveyed to the column.

6. Process according to either of Claims 4 and 5, **characterized in that** the flow (6) obtained at the bottom of the column (C1), if appropriate at the bottom of the column (S1), is conveyed onto a plate situated in the lower half of the column (C3) and that the operating point of the said column (C3) is carefully chosen so as to obtain:
- at the top, a flow (7) composed:
- predominantly, i.e. at least 95% by weight, of acrylic acid;
- the remainder being composed of the heavy compounds: maleic anhydride, furfuraldehyde, benzaldehyde and traces of ditolyl ether; and
- at the bottom, a flow (8) composed of:
- predominantly, i.e. at least 95% by weight, of ditolyl ether and of the heavy impurities;
- the remainder being composed of acrylic acid.

7. Process according to one of Claims 4 to 6 **characterized in that** the flow (10) rich in maleic anhydride and heavy impurities is drawn-off from the side of the column (C4), on an intermediate plate situated above the feed between the lower quarter and the upper quarter of this column, at a temperature chosen so as to obtain a flow (10) with a concentration at least equal to 20% by weight of impurities with boiling temperatures between that of acrylic acid and that of ditolyl ether or of the lighter of the ditolyl ether isomers used as a mixture.

8. Process according to one of Claims 4 to 7, **characterized in that** the flow (9) distilled at the top of the column (C4), which comprises:
- predominantly, i.e. at least 90% by weight, acrylic acid;
- the remainder being composed of impurities with higher boiling temperatures, is conveyed to the column (C3) at the level of the main feed of this column or at a level situated above this feed.

9. Process according to one of Claims 4 to 8, **characterized in that**, before recycling, at the top of the column (C1), the flow (1) obtained at the bottom of column (C4), the said flow or a portion of the said flow is freed (in 11) from its heavy impurities with boiling temperatures greater than that of ditolyl ether, for example by techniques comprising distillation or extraction with the aid of a solvent, optionally used in addition to a thermal dissociation treatment which may or may not involve a catalyst.

10. Process according to one of Claims 4 to 9, **characterized in that** the distillation is carried out:
- in the column (C3), under a pressure of 2.66 × 10³ - 3.33 × 10⁴ Pa, at a top temperature of 40 - 120°C and at a bottom temperature of 120 - 230°C; and
- in the column (C4), under a pressure of 2.66 × 10³ - 3.33 × 10⁴ Pa, at a top temperature of 40 - 120°C, at a bottom temperature of 120 - 230°C and at a side draw-off temperature of 40 - 180°C.

## Patentansprüche

1. Verfahren zur Reinigung von durch katalytische Oxidation von Propylen erhaltener Acrylsäure, gemäß dem man die Acrylsäure durch Gegenstromwäsche der Reaktionsgase mit mindestens einem schweren hydrophoben Absorptionslösungsmittel extrahiert und dann aus der danach erhaltenen Lösung die gereinigte Acrylsäure gewinnt, **dadurch gekennzeichnet, daß** man als schweres hydrophobes Absorptionslösungsmittel Ditolylether in Form eines einzelnen Isomers oder eines Isomerengemischs verwendet.

2. Verfahren nach Anspruch 1, **dadurch gekennzeichnet, daß** man die Reinigung der Acrylsäure nach dem Extraktionsschritt bei Beteiligung von Destillationen in Gegenwart eines Polymerisationsinhibitors oder eines Gemischs von Polymerisationsinhibitoren, der bzw. das leichter ist als Ditolylether und insbesondere einen Siedepunktsunterschied gegenüber Ditolylether von mehr als 15°C und vorzugsweise mehr als 20°C aufweist.

3. Verfahren nach Anspruch 2, **dadurch gekennzeichnet, daß** man den oder die Polymerisationsinhibitoren unter p-Methoxyphenol, 2,6-Di-tert.-butyl-p-kresol und 2,4-Dimethyl-6-tert.-butylphenol auswählt.

4. Verfahren nach einem der Ansprüche 1 bis 3,
**dadurch gekennzeichnet, daß** man:
- den im Sumpf der Extraktionskolonne (C1), in der die Extraktion der Acrylsäure durch Gegenstromwäsche der Reaktionsgase mit Ditolylether erfolgt, erhaltenen Strom (6), der Ditolylether, die gewünschte Acrylsäure und Verunreinigungen, hauptsächlich Verunreinigungen mit höheren Siedetemperaturen als Acrylsäure, enthält, in einer Destillationskolonne (C3) unter solchen Bedingungen destilliert, daß man am Kopf der Kolonne (C3) einen sehr reinen Acrylsäurestrom (7) erhält, wobei man Acrylsäure in den Sumpf (8) gehen läßt;
- den Sumpfstrom (8) aus der Kolonne (C3) als Zufuhr dem unteren Teil einer Destillationskolonne (C4) zuführt, aus welcher man an einem zwischen der Zufuhr und dem Kolonnenkopf gelegenen Boden einen an Maleinsäureanhydrid und Verunreinigungen mit zwischen den Siedepunkten von Acrylsäure und Ditolylether bzw. dem leichteren der im Gemisch verwendeten Ditolyletherisomere liegenden Siedepunkten reichen Strom (10) seitlich abzieht;
- am Kopf der Kolonne (C4) einen acrylsäurereichen Strom (9) abdestilliert, der vorteilhafterweise wieder der Kolonne (C3) zugeführt werden kann; und
- im Sumpf der Kolonne (C4) einen das schwere Extraktionslösungsmittel bzw. die schweren Extraktionslösungsmittel und schwere Verunreinigungen, deren Siedepunkte über dem Siedepunkt von Ditolylether bzw. dem leichteren der im Gemisch verwendeten Ditolyletherisomere liegt, enthaltenden Strom (1) gewinnt, welchen man zur Extraktion der in den Reaktionsgasen enthaltenen Acrylsäure zum Kopf der Kolonne (C1) zurückführt.

5. Verfahren nach Anspruch 4, **dadurch gekennzeichnet, daß** man den im Sumpf der Kolonne (C1) erhaltenen Strom am Kopf einer Kolonne (S1), die als herkömmliche Destillationskolonne mit Sumpfverdampfer oder als im Sumpf mit einem Gas, wie Luft, einem Inertgas oder einem am Kopf der Kolonne (C1) gewonnenen nichtkondensierten Gasgemisch oder einer Kombination davon gespeiste Strippsäule arbeiten kann, von einem Teil seiner leichten Restverunreinigungen, wie Essigsäure und Wasser, befreit, wobei man den am Kopf der Kolonne (S1) erhaltenen, noch Acrylsäure enthaltenden Gasstrom vorteilhafterweise wieder der Kolonne zuführt.

6. Verfahren nach Anspruch 4 oder 5, **dadurch gekennzeichnet, daß** man den im Sumpf der Kolonne (C1) und gegebenenfalls am Boden der Kolonne (S1) erhaltenen Strom (6) einem Boden in der unteren Hälfte der Kolonne (C3) zuführt und den Betriebspunkt der Kolonne (C3) so aussucht, daß man:
- am Kopf einen Strom (7), der
- hauptsächlich, d.h. zu mindestens 95 Gew.-%, aus Acrylsäure besteht,
- wobei der Rest aus schweren Verbindungen besteht: Maleinsäureanhydrid, Furfuraldehyd, Benzaldehyd und Ditolyletherspuren;
- im Sumpf einen Strom (8), der
- hauptsächlich, d.h. zu mindestens 95 Gew.-%, aus Ditolylether und schweren Verunreinigungen besteht,
- wobei der Rest aus Acrylsäure besteht;
erhält.

7. Verfahren nach einem der Ansprüche 4 bis 6, **dadurch gekennzeichnet, daß** man aus der Kolonne (C4) den an Maleinsäureanhydrid und schweren Verunreinigungen reichen Strom (10) an einem oberhalb der Zufuhr zwischen dem unteren Viertel und dem oberen Viertel dieser Kolonne gelegenen Zwischenboden bei einer Temperatur seitlich abzieht, welche so gewählt ist, daß man einen Strom (10) mit einer Konzentration an Verunreinigungen mit zwischen den Siedepunkten von Acrylsäure und Ditolylether bzw. dem leichteren der im Gemisch verwendeten Ditolyletherisomere liegenden Siedepunkten von mindestens 20 Gew.-% erhält.

8. Verfahren nach einem der Ansprüche 4 bis 7, **dadurch gekennzeichnet, daß** man den am Kopf der Kolonne (C4) abdestillierten Strom (9), der
- hauptsächlich, d.h. mindestens 90 Gew.-%, Acrylsäure enthält,
- wobei der Rest aus Verunreinigungen mit höheren Siedetemperaturen besteht;
der Kolonne (C3) auf Höhe der Hauptzufuhr dieser Kolonne oder unterhalb dieser Zufuhr zuführt.

9. Verfahren nach einem der Ansprüche 4 bis 8, **dadurch gekennzeichnet, daß** man vor der Rückführung des im Sumpf der Kolonne (C4) erhaltenen Stroms (1) zum Kopf der Kolonne (C1) diesen Strom oder einen Teil davon (bei 11) von seinen schweren Verunreinigungen mit höheren Siedetemperaturen als Ditolylether befreit, beispielsweise durch Destillations- oder Lösungsmittelextraktionstechniken, gegebenenfalls als Ergänzung einer thermischen Dissoziationsbehandlung mit oder ohne Katalysator.

10. Verfahren nach einem der Ansprüche 4 bis 9, **dadurch gekennzeichnet, daß** man die Destillation:
- in der Kolonne (C3) unter einem Druck von 2,66 x 10³ - 3,33 x 10⁴ Pa, einer Kopftemperatur von 40 - 120°C und einer Sumpftemperatur von 120 - 230°C und
- in der Kolonne (C4) unter einem Druck von 2,66 x 10³ - 3,33 x 10⁴ Pa, einer Kopftemperatur von 40 - 120°C, einer Sumpftemperatur von 120 - 230°C und einer Seitenabzugstemperatur von 40 - 180°C
durchführt.
